Europäisches Patentamt

European Patent Office

Office européen des brevets

(19)

(11) Publication number : **0 484 106 A1**

(12) # EUROPEAN PATENT APPLICATION

(21) Application number : 91309986.7

(22) Date of filing : 29.10.91

(51) Int. Cl.⁵ : **A61K 9/20,** A61K 31/52, A61K 33/06, A61K 33/08, A61K 33/12

Amended claim in accordance with Rule 86 (2) EPC for the following Contracting State: ES, GR.

(30) Priority : 01.11.90 US 608075

(43) Date of publication of application : 06.05.92 Bulletin 92/19

(84) Designated Contracting States :
AT BE CH DE DK ES FR GB GR IT LI LU NL SE

(71) Applicant : **MERCK & CO. INC.**
**126, East Lincoln Avenue P.O. Box 2000**
**Rahway New Jersey 07065-0900 (US)**

(72) Inventor : **Colegrove, George T.**
**5238 Fontaine Street**
**San Diego, California 92120 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **Controlled release formulations.**

(57)   Antacid compound salts of sodium and magnesium ions, e.g. magnesium oxide, magnesium hydroxide, magnesium trisilicate, magnesium phosphate, and trisodium phosphate, are combined with water soluble alginates, e.g. sodium, potassium and magnesium alginate in a controlled release tablet containing an active ingredient, to produce a tablet having an improved hydration environment for the alginate. The improved hydration environment allows the alginate particles to swell in gastric fluid and produce a thicker and more uniform layer through which the active ingredient must diffuse, thereby decreasing the rate of release.

FIG. I

EP 0 484 106 A1

## BACKGROUND OF THE INVENTION

The invention relates to controlled long-acting release pharmaceutical formulations containing an active therapeutic agent and a carrier base material. More specifically, this invention relates to tablets of a controlled long-acting pharmaceutical formulation containing an active therapeutic agent and a carrier base material.

Long-acting products are widely marketed in the pharmaceutical field and are now a significant factor in the administration of a variety of active pharmaceutical agents. The advantages of such long-acting or sustained release products are well understood and a very substantial industry has developed around these products. Sustained release products permit various medications to be administered for uniform and continuous release over a prolonged period of time, thereby achieving a particular blood level of active ingredient for whatever time is thought to be advantageous to the patient. Such administration obviates the necessity of frequent administration of active ingredient and avoids the problems inherent in insuring timely and repetitive consumption of pharmaceutical product by the patient. It is possible to achieve stable blood levels of a variety of active therapeutic agents and thereby control a variety of physiological conditions. It also reduces or possibly eliminates toxic or side effects which are caused by frequent administration of active ingredients through the peaks and valleys of blood levels caused by multiple ingestion of medication.

In the production of controlled or sustained release tablets by direct compression of dry powders, a water soluble thickener is commonly used to provide a matrix for the tablet. The thickener hydrates rapidly on the surface of the tablet and swells to a gelatinous consistency through which the drug must diffuse, thus decreasing the rate of diffusion of the active drug. Alginates tend to swell in gastric fluid and produce a gelled layer within the first few millimeters of the tablet which retards diffusion of the drug. Sodium alginate is often used to form the gel matrix. However, it does not dissolve well at pH's as low as those encountered in gastric fluid and, therefore, does not hydrate well in the gastric fluid environment. The following patents describe various controlled release systems which include alginates.

Wheatley et al., US Patent 4,933,185, describes a system for controlled release of biologically active substances such as proteins. The system contains the active substance and a polysaccharide degrading enzyme encapsulated within a microcapsule. The microcapsule has an inner polysaccharide polymer core and an outer ionically interacting skin. One of the exemplary polysaccharides is alginate.

Horder et al., US Patent 4,842,866, describes a controlled release system which contains an active ingredient, sodium alginate, and a calcium-sodium alginate complex. The amount of calcium used in the calcium-sodium alginate complex is precisely controlled, and the complex is self-gelling.

Connick, US 4,401,456, describes a process for producing alginate gel beads containing an herbicide material.

Scher, US 4,053,627, describes a method for controlling the release of an insect juvenile hormone from chemical degradation by incorporating the hormone into gel discs containing water soluble algin, a calcium salt for gelatinization, a solubilizing agent, and a biocide.

The present invention is a controlled or sustained release tablet comprising an antacid material which increases the pH of the environment surrounding the alginate and induces improved hydration. Improved alginate particle swelling produces a thicker and more uniform layer through which the drug must diffuse, thereby decreasing the release rate.

## SUMMARY OF THE INVENTION

The invention is a tablet for controlled release of a biologically active substance comprising a biologically active substance, a water soluble alginate, and a magnesium or sodium antacid. The amount of alginate present in the tablet is between about 5 and 50%, preferably 15 and 30%, by weight and the amount of antacid present in the tablet is between about 1 and 10%, preferably 2.5-7.5%, by weight.

## BRIEF DESCRIPTION OF THE FIGURES

Figure 1 shows dissolution of theophylline in simulated gastric fluid in various 20% Keltone HV controlled release tablets.

Figure 2 shows dissolution of theophylline in simulated intestinal fluid following 2 hours exposure to simulated gastric fluid in various 20% Keltone HV controlled release tablets.

## DETAILED DESCRIPTION OF THE INVENTION

Preferred antacids which are suitable for use in tablets of the present invention are sodium antacids such as trisodium phosphate and magnesium antacids. More preferred antacids are magnesium antacids, e.g. magnesium oxide, magnesium hydroxide, magnesium trisilicate and magnesium phosphate.

Antacids which upon neutralization with gastric fluid produce cations that react with alginate and prevent the solubility or swelling of the alginate are not desirable. Calcium and aluminum ions both produce strong gels with alginates and are therefore inadequate. Bismuth antacids are also undesirable.

The term alginate refers to alginic acid and the salts thereof. Alginic acid, derived primarily from kelp, is a commercially available product, e.g., KELACID® (Kelco Div., Merck & Co., Inc.). The salts include appropriate metal salts e.g. alkali metal, alkaline earth metal, ammonium salts, and organic derivatives e.g. alkylene gylcol, propylene glycol and the like. The salts are preferrably water soluble. The preferred salts are sodium, potassium, magnesium, ammonium and propylene glycol alginate. KELTONE HV® is a high viscosity sodium alginate available from Kelco Div., Merck & Co., Inc.

Among the active therapeutic agents which are useful in the present invention are sedatives, vitamins, anti-inflammatory agents, vasodilators, stimulants, relaxants, suppressants, and many other types of therapeutic agents. Other active ingredients are, for example, isosorbide dinitrate or mononitrate (employed in the treatment of angina pectoris), theophylline (employed in the treatment of asthma), nitroglycerin, ibuprofen, and acetaminophen.

The antacids can be dry blended with other materials of the tablet composition and compressed directly using common tableting equipment.

In preparing the pharmaceutical compositions of the invention, the desired ratio of active ingredient and tablet forming material is introduced into a mixing vessel. Ingredients which may be introduced into the mixing vessel are, for example, fillers, drying agents, lubricants, coloring agents, starch, and other materials well known in the art. Thereafter the base mixture is typically agitated and mixed for from 20 to 40 minutes and usually from 30 to 40 minutes to achieve uniformity of the active ingredients with the base mixture. Mixing equipment may be, for example, a Day mixer or a Pony mixer.

After a uniform mixture has been obtained, it is transferred to a shaping and compressing step as is well known in the art. The equipment used for such steps may be, for example, Stokes or Colton rotary machines or other tablet compressing machines.

A typical procedure for combining the various ingredients and making the various tablets in the Examples below, is as follows:

The base ingredients are mixed for about 20 minutes in a Day powder mixer or a Pony mixer. Active ingredient is added to the base mixture and the mixture is again mixed for about 30 minutes, adding lubricants. The ingredients are conventionally compressed into tablets of varying shape, including capsule and round shape. For example round tablets having 3/8″ punch size and 15-20 kg hardness are prepared. In the examples presented below, the potency of the tablet is 100 mg active ingredient per 400 mg tablet.

EXAMPLE 1 (Standard)

Following the general procedure described above, a controlled release composition containing the following ingredients was prepared:

| Ingredients | wt. % | mg/400 mg tablet |
|---|---|---|
| Keltone HV | 20% | 80 |
| Theophylline | 25 | 100 |
| Lactose | 54 | 216 |
| Magnesium stearate | 1 | 4 |

Ingredient amounts are shown in dry weight form.

EXAMPLE 2

Following the general procedure described in Example 1, but including magnesium oxide, a controlled release composition containing the following ingredients was prepared:

| Ingredient | wt. % |
|---|---|
| Keltone HV | 20% |
| Magnesium Oxide | 10 |

| Theophylline | 25 |
|---|---|
| Lactose | 44 |
| Magnesium stearate | 1 |

EXAMPLE 3

Following the general procedure described in Example 1, but including trisodium phosphate, a controlled release composition containing the following ingredients prepared:

| Ingredients | wt. % |
|---|---|
| Keltone HV | 20% |
| Trisodium phosphate | 10 |
| Theophylline | 25 |
| Lactose | 44 |
| Magnesium stearate | 1 |

EXAMPLE 4, 5, and 6

Following the general procedure of Example 1, the following tablets were prepared:

| | Example (wt. %) | | |
|---|---|---|---|
| Ingredients | 4 | 5 | 6 |
| Keltone HV | 20% | 20 | 20 |
| Magnesium Oxide | 1 | 2.5 | 5 |
| Theophylline | 10 | 10 | 10 |
| Lactose | 68 | 66.5 | 64 |
| Magnesium stearate | 1 | 1 | 1 |

Release of each tablet is measured by testing in simulated gastric fluid at pH 1.3 in a Milton Roy tablet dissolution apparatus for three hours at 37°C. Release is determined spectrophotometrically and plotted versus time.

Release is also measured by placing the tablet in simulated gastric fluid at low pH (e.g. 0.1 N HC1, 0.2% NaC1 @ pH 1.3) for two hours followed by testing in simulated intestinal fluid without enzyme (e.g. phosphate buffer solution @ pH 7.5 as prepared in USP XXI p. 124 as "Test Solutions").

EXAMPLE 7

Dissolution of theophylline in simulated gastric fluid

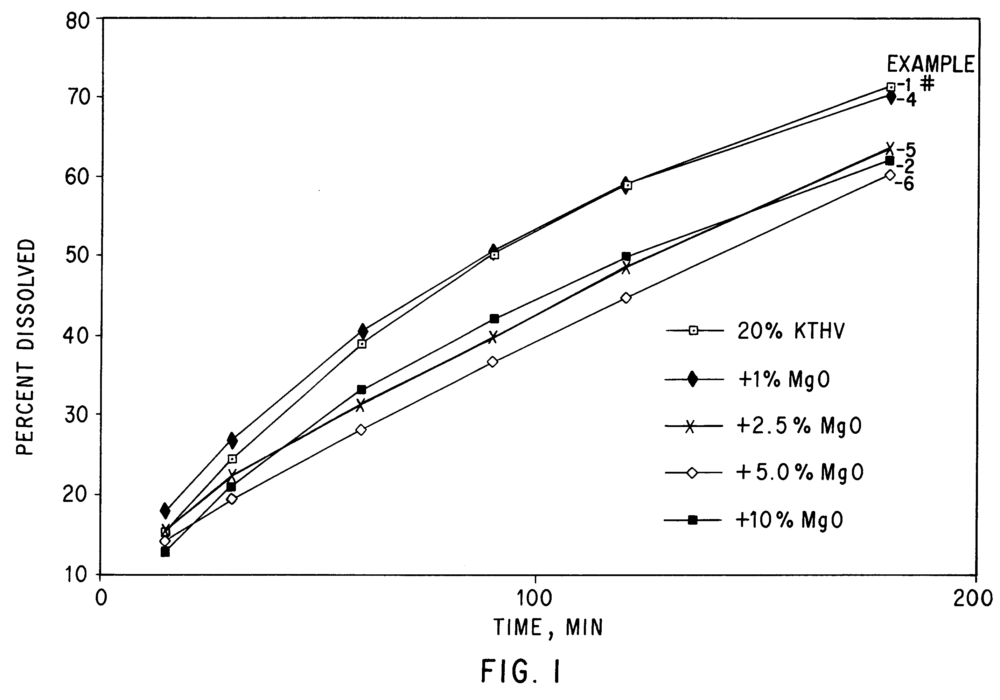

Results of controlled release testing are shown in Figure 1. Figure 1 shows that release of the active ingredient is delayed when magnesium oxide is present. The following data representations are made:

| | | | Example |
|---|---|---|---|
| ▣ | 20% | Keltone HV | 1 |
| ◆ | 1% | MgO | 4 |
| ◘ | 2.5% | MgO | 5 |
| ◈ | 5.0% | MgO | 6 |
| ■ | 10% | MgO | 2 |

4

EXAMPLE 8

Release of theophylline in simulated intestinale fluid after 2 hours in simulated gastric fluid

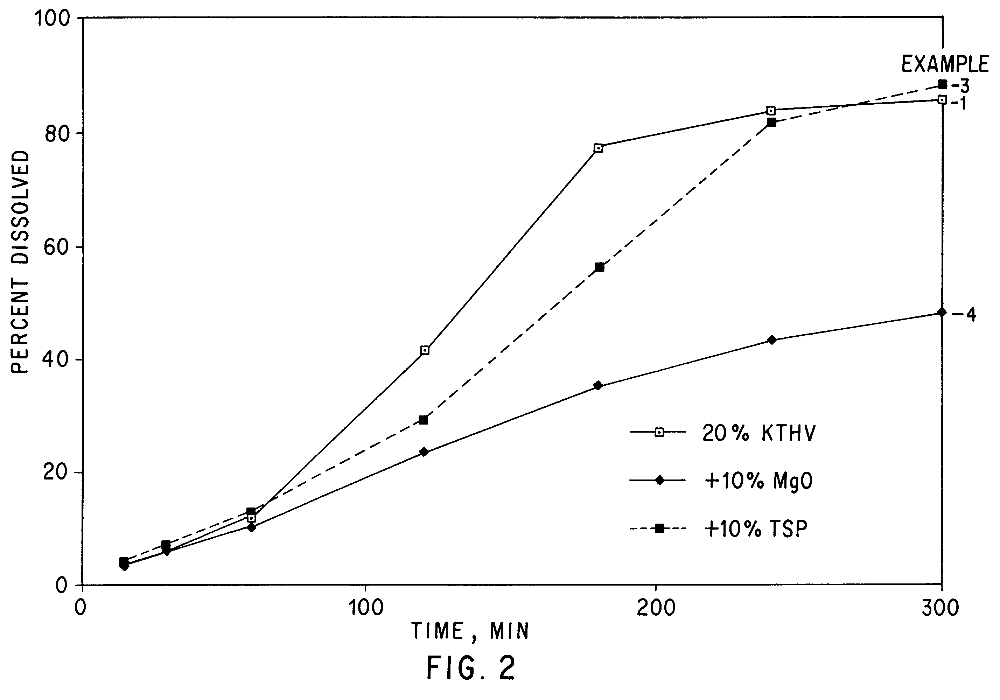

Results of controlled release testing are shown in Figure 2. Figure 2 shows that release of the active ingredient is delayed when either magnesium oxide or trisodium phosphate is present.
The following data representations are made:

|   |   |   | **Example** |
|---|---|---|---|
| ▣ | 20% | Keltone HV | 1 (standard) |
| ◆ | 10% | MgO | 4 |
| ▣ | 10% | Trisodium phosphate | 3 |

The data shows surprisingly and unexpectedly that controlled release was enhanced using sodium or magnesium salts. Viscous layers surrounding controlled release tablets known in the art dissolve rapidly at neutral pH. In the presence of the antacid compound, the viscous layer is maintained for a much longer period of time, thereby reducing the release rate of the active ingredient.

## Claims

1. A tablet for controlled release of a biologically active substance comprising:
   (a) a biologically active substance;
   (b) between about 5 and 50% by weight of water soluble alginate, and
   (c) between about 1 and 10% by weight of a magnesium or sodium antacid.

2. The tablet of claim 1 wherein the biologically active substance is theophylline.

3. The tablet of claim 1 wherein the water soluble alginate is a sodium alginate.

4. The tablet of claim 1 wherein the antacid is selected from the group consisting of sodium antacids.

5. The tablet of claim 1 wherein the antacid is selected from the group consisting of magnesium antacids.

6. The tablet of claim 4 wherein the sodium antacid is trisodium phosphate.

7. The tablet of claim 5 wherein the magnesium antacid is selected from the group consisting of magnesium oxide, magnesium hydroxide, magnesium trisilicate and magnesium phosphate.

8. The tablet of claim 1 wherein the magnesium or sodium antacid is present in an amount from 2.5-7.5%

9. The tablet of claim 1 wherein the alginate is present in an amount from 15-30%

**Claims for the following Contracting States: ES, GR**

1. A process for preparing a tablet for controlled release of a biologically active substance comprising:
   (a) a biologically active substance;
   (b) between about 5 and 50% by weight of water soluble alginate, and
   (c) between about 1 and 10% by weight of a magnesium or sodium antacid, which process comprises the following steps:
      1. mixing the base ingredients;
      2. adding the active ingredient to the base mixture with mixing; and
      3. compressing the mixture into tablets.

2. A process for preparing the tablet of claim 1 wherein the active ingredient is theophylline.

3. A process for preparing the tablet of claim 1 wherein the water soluble alginate is a sodium alginate.

4. A process for preparing the tablet of claim 1 wherein the antacid is a sodium antacid.

5. A process for preparing the tablet of claim 1 wherein the antacid is a magnesium antacid.

6. A process for preparing the tablet of claim 4 wherein the sodium antacid is trisodium phosphate.

7. A process for preparing the tablet of claim 5 wherein the magnesium antacid is selected from the group consisting of magnesium oxide, magnesium hydroxide, magnesium trisilicate and magnesium phosphate.

8. A process for preparing the tablet of claim 1 wherein the magnesium or sodium antacid is present in an amount from 2.5-7.5%.

9. A process for preparing the tablet of claim 1 wherein the alginate is present in an amount from 15-30%.

FIG. I

FIG. 2

EP 0 484 106 A1

**European Patent Office**

# EUROPEAN SEARCH REPORT

Application Number

EP 91 30 9986

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 199 560 (L. GYARMATI)<br>* Claims; column 11, example 7 *<br>--- | 1,3,5,7 | A 61 K 9/20<br>A 61 K 31/52<br>A 61 K 33/06<br>A 61 K 33/08<br>A 61 K 33/12 |
| A | BE-A- 802 700 (BIOREX)<br>* Claims; page 4, examples *<br>--- | 1,3,5,7 | |
| A | FR-A-2 636 532 (GLAXO)<br>* Claims 1,6-7,10,20-21 *<br>--- | 1,3,5,7 | |
| A | WO-A-9 011 757 (DEPOMED)<br>* Claims 1-2,8-9; page 3, lines 30-37; page 5, line 11 *<br>--- | 1,3,8-9 | |
| A | EP-A-0 153 836 (HEALTH PRODUCTS)<br>* Claims 1-3,9-10,14-15; page 3, lines 29-31; page 4, lines 1-10; page 7, example III; page 8, example V *<br>----- | 1,3,5,7-9 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 14-01-1992 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P0401)